# EUROPEAN PATENT APPLICATION

(11) **EP 3 903 762 A1**
(43) Date of publication of application: **03.11.2021**
(21) Application number: 19903104.8
(22) Date of filing: 19.12.2019
(51) Int. Cl.: A61K 8/06, A61K 8/37, A61K 8/67, A61K 8/81

(54) **RETINOL-CONTAINING OIL-IN-WATER TYPE EMULSIFIED COSMETIC MATERIAL**

(30) Priority: 28.12.2018 JP 2018247049
(71) Applicant: Shiseido Company, Ltd., Chuo-ku Tokyo 104-0061 (JP)
(72) Inventor: YABUSAKI, Yusuke, Tokyo 104-0061 (JP)
(74) Representative: Ter Meer Steinmeister & Partner
(86) International application number: PCT/JP2019/049789
(87) International publication number: WO 2020/137785

(57) **Abstract**

An objective of the present invention is to provide a retinol-containing oil-in-water emulsion cosmetic that has excellent emulsion stability while stably retaining retinol, that also has a good fitting sensation when applied, and that has a watery and fresh feeling in use. The present invention pertains to an oil-in-water emulsion cosmetic containing (a) retinol; (b) an alkyl-modified carboxyvinyl polymer; and (c) an oil, wherein a blended amount of the (c) oil is 10% to 40% by mass relative to a total amount of the cosmetic, and the (c) oil contains at least 50% by mass of an ester oil relative to a total amount of the oil.

## Description

### TECHNICAL FIELD

The present invention relates to an oil-in-water emulsion cosmetic that contains retinol. More specifically, the present invention relates to a cosmetic that has excellent emulsion stability while stably retaining retinol for a long period of time, that also has a good fitting sensation when applied, and that achieves a watery and fresh feeling in use, particularly to an oil-in-water emulsion cosmetic that is suitable for use around the eyes.

### BACKGROUND ART

Vitamins in the vitamin A family, such as retinol, are known to be substances having various types of bioactivity. For example, many cosmetic products that are marketed contain vitamins in the vitamin A family, such as retinol, as beauty components exhibiting wrinkle alleviation effects. However, retinol is an extremely unstable substance, and tends to he easily degraded by heat, light, air (i.e., oxygen) and the like.

Therefore, in order to suppress the deterioration of retinol due to oxidation, it is common to blend retinol together with an antioxidant. In particular, phenolic antioxidants such as tocopherol and dibutyl hydroxytoluene (BHT), which have excellent antioxidant effects in small amounts, are commonly used. However, since phenolic antioxidants such as tocopherol and BHT prevent the oxidation of coexisting substances by being oxidized themselves, there were cases in which problems occurred, such as the antioxidant effect weakening over time, as well as discoloration and odor generation. Additionally, when a large amount of an antioxidant was blended in anticipation of deterioration occurring over time, there were problems in which the antioxidant precipitated, particularly when stored at low temperatures.

Therefore, various improvements have been made to allow retinol to be stably retained, even when the antioxidant is reduced or not blended.

For example, the cosmetic composition described in Patent Document 1 is a cosmetic oil-in-water emulsion composition containing a polymer emulsifier and a retinoid solubilized in a fluid oil. Patent Document 1 describes that the stability of the retinoid was improved by minimizing the exposure of the retinoid to water by the polymer emulsifier minimizing the diffusion of water into the oil phase. However, the fluid oils whose effects were verified were silicone oils and dicaprylyl ether, the blended amounts of which were small (5% or less). Thus, the texture when applied to skin was unsatisfactory.

Patent Document 2 describes an O/W emulsion composition containing an oil-soluble active substance such as retinol, wherein an alkyl-modified carboxyvinyl polymer is blended as substantially the only emulsifier, the composition pH is adjusted to be 4.0 to 8.0, and the average particle size of the emulsion particles is set to be 2 to 30 µm, thereby allowing the safety and the storage stability of the oil-soluble active substance to be improved while maintaining emulsion stability. However, the oils blended in the composition of Patent Document 2 were hydrocarbon oils such as squalane, silicone oils and higher alcohols. Thus, the texture was poor, as in the composition of Patent Document 1.

The fact that alkyl-modified carboxyvinyl polymers used in Patent Documents 1 and 2 cooperate with liquid higher alcohols at room temperature and thereby improve the emulsion stability (Patent Document 3) and the fact that polyethylene glycol and/or polypropylene glycol are effective in stabilizing retinol (Patent Document 4) are conventionally known. However, no examples in which the texture is improved while maintaining the stability and the emulsion stability of retinol are known. In particular, in cosmetics that are applied around the eyes, the fitting sensation when applied and the feeling in use, such as the wateriness and the freshness, are important, in view of which retinol-containing cosmetics having such excellent texture are sought.

### RELATED ART

### PATENT DOCUMENTS

Patent Document 1: JP 2004-513959 A
Patent Document 2: JP 2013-241406 A
Patent Document 3: JP H8-126831 A
Patent Document 4: JP H6-32710 A

### SUMMARY OF THE INVENTION

### PROBLEM TO BE SOLVED BY THE INVENTION

Therefore, the problem addressed by the present invention is to provide a retinol-containing oil-in-water emulsion cosmetic that has excellent emulsion stability while stably retaining retinol, that has a good fitting sensation when applied, and that has a watery and fresh feeling in use.

### MEANS FOR SOLVING THE PROBLEM

The present inventors performed diligent research towards solving the aforementioned problem, as a result of which they discovered that a cosmetic that has excellent emulsion stability while stably retaining retinol, that also has a good fitting sensation when applied to skin, and that provides a watery and fresh feeling in use can be obtained, even using substantially no antioxidants, by adjusting the blended amount of oils and the percentage of the total weight of oils occupied by ester oils so as to be within prescribed ranges in an oil-in-water emulsion cosmetic containing retinol and alkyl-modified carboxyvinyl polymer as active ingredients.

Specifically, the present invention provides an oil-in-water emulsion cosmetic containing:
(a) retinol;
(b) an alkyl-modified carboxyvinyl polymer; and
(c) an oil; wherein
a blended amount of the (c) oil is 10% to 40% by mass relative to a total amount of the cosmetic, and the (c) oil contains at least 50% by mass of an ester oil relative to a total amount of the oil.

### EFFECTS OF THE INVENTION

The oil-in-water emulsion cosmetic of the present invention can stably retain retinol for a long period of time, has a good fitting sensation when applied while also maintaining high emulsion stability, and provides a watery and fresh feeling in use.

Additionally, since no solid oils need to be contained as oils, there is no need for heating during production. Furthermore, since substantially no antioxidant needs to be contained therein, there is no risk of discoloration, odor generation or precipitation over time.

### MODES FOR CARRYING OUT THE INVENTION

The oil-in-water emulsion cosmetic (hereinafter also referred to simply as "cosmetic") of the present invention contains, as essential components, (a) retinol; (b) an alkyl-modified carboxyvinyl polymer; and (c) an oil of which at least 50% by mass is an ester oil. Hereinafter, the respective components constituting the emulsion cosmetic of the present invention will be explained in detail.

### (a) Retinol

The "retinol" (component (a)) in the cosmetic of the present invention includes retinol and derivative thereof. As retinol derivatives, retinol palmitate, retinol acetate, Cylasphere retinol, tocopheryl retinoate and the like are commonly known. In the present invention, non-derivative retinol (also known as pure retinol) is preferably used. In the present specification, "retinol" shall refer to pure retinol. Retinol is a substance that is extremely unstable, but has extremely strong effects in terms of wrinkle alleviation in comparison with derivatives. However, by blending retinol into the cosmetic of the present invention, it can be stably retained for a long period of time even without adding an antioxidant.

The blended amount of retinol in the cosmetic of the present invention should be 0.001% to 0.5% by mass, preferably 0.01% to 0.1% by mass relative to the total amount of the emulsion cosmetic of the present invention. In this case, if the blended amount is less than 0.001% by mass, then the effects obtained by blending retinol cannot be adequately achieved. Conversely, if more than 0.5% by mass is blended, then there are cases in which the skin becomes irritated.

### (b) Alkyl-modified carboxyvinyl polymer

The "alkyl-modified carboxyvinyl polymer" (component (b)) in the cosmetic of the present invention includes alkyl-modified carboxyvinyl polymer and salts thereof.

The alkyl groups in the alkyl-modified carboxyvinyl polymer used in the present invention may be linear or branched. The number of carbon atoms constituting the alkyl groups is not particularly limited, but may, for example, be 8 to 35, preferably 10 to 30.

As the alkyl-modified carboxyvinyl polymer of the present invention, it is possible to use, for example, a copolymer obtained by copolymerizing an olefinic unsaturated carboxylic acid monomer with a (meth)acrylate monomer modified with a long-chain alkyl group having 8 to 35 (preferably 10 to 30) carbon atoms. Typical examples include copolymers obtained by copolymerizing 1% to 50% by weight of a long-chain alkyl (meth)acrylate monomer with 50% to 99% by weight of an olefinic unsaturated carboxylic acid monomer. Depending on the situation, in order to adjust the properties of the copolymer, a copolymer having a crosslinked structure polymerized by further blending an olefinic polyfunctional monomer may be used (see, for example, JP 2002-193731 A).

As the long-chain alkyl (meth)acrylate monomer constituting the alkyl-modified carboxyvinyl polymer, a monomer represented by the following general formula (1) can be used. 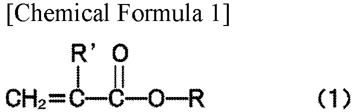 (R is a linear or branched alkyl group having 8 to 35, preferably 10 to 30 carbon atoms, and R' is hydrogen or a methyl group.)

Specific examples include one or more monomers selected from the group consisting of alkyl acrylates having 8 to 35, preferably 10 to 30 carbon atoms, such as decyl acrylate, lauryl acrylate, palmityl acrylate, stearyl acrylate and behenyl acrylate, and methacrylates corresponding thereto.

Additionally, examples of olefinic unsaturated carboxylic acid monomers that can be copolymerized with the long-chain alkyl (meth)acrylate monomer include one or more monomers selected from the group consisting of polymerizable compounds including an olefinic double bond and at least one carboxylic acid group, such as acrylic acid, methacrylic acid, α-chloro-acrylic acid, α-phenyl acrylic acid, sorbic acid, cinnamic acid, maleic acid, fumaric acid and maleic anhydride. Among the above, acrylic acid, which is easily available and which has excellent polymerization capacity, is preferred.

As the copolymer formed from a long-chain alkyl (meth)acrylate and an olefinic unsaturated carboxylic acid monomer, it is possible to use a copolymer having a crosslinked structure obtained by further adding and polymerizing, to the long-chain alkyl (meth)acrylate and the olefinic unsaturated carboxylic acid monomer, an olefinic polyfunctional monomer such as methylene bisacrylamide, allyl acrylate, methallyl methacrylate, divinyl ether, allyl sucrose, trimethylolpropane triacrylate, trimethylolpropane trimethacrylate and glyceryl triacrylate, in order to adjust the properties of the copolymer. The added amount of the olefinic polyfunctional monomer should preferably be 0.01% to 4.0% by mass, more preferably 0.2% to 1.0% by mass relative to the total amount of the long-chain alkyl (meth)acrylate monomer and the olefinic unsaturated carboxylic acid monomer. The copolymers having these crosslinked structures may, for example, be produced by the method described in JP S51-6190 A or the like.

The average molecular weight of the (b) alkyl-modified carboxyvinyl polymer used in the present invention is not particularly limited, but should preferably be approximately 500,000 to approximately 5,000,000.

The (b) alkyl-modified carboxyvinyl polymer used in the cosmetic of the present invention should preferably be of the type known as "(acrylates/(C10-30) alkyl acrylate) crosspolymer" or "(acrylates/(C12-22) alkyl methacrylate) copolymer" in terms of cosmetic ingredient nomenclature. These polymers are commercially available under the trade names PEMULEN TR-1 (manufactured by Lubrizol Advanced Materials), PEMULEN TR-2 (manufactured by Lubrizol Advanced Materials), CARBOPOL 1342 (manufactured by Lubrizol Advanced Materials), CARBOPOL ETD2020 (manufactured by Lubrizol Advanced Materials) and Allianz OPT (manufactured by Ashland), and these commercially available products may be used.

Although the blended amount of the (b) alkyl-modified carboxyvinyl polymer in the cosmetic of the present invention is not particularly limited, it should normally be 0.01% to 4.0% by mass, preferably 0.01% to 2.0% by mass, more preferably 0.01% to 1.0% by mass, and even more preferably 0.01% to 0.5% by mass relative to the total amount of the emulsion cosmetic.

The alkyl-modified carboxyvinyl polymer may be of one type or may be a mixture of two or more types.

### (c) Oil

In the cosmetic of the present invention, the blended amount of the oil (component (c)) is 10% to 40% by mass relative to the total amount of the cosmetic, and the oil is adjusted so as to contain at least 50% by mass of an ester oil relative to the total amount of the oil, thereby achieving a fitting sensation when applied and a watery feeling in use, in addition to stability.

The "ester oil" blended into the oil (component (c)) in the present invention is not particularly limited as long as it is an ester oil that can be blended into a cosmetic. In the present specification, the "ester oil" includes compounds in which an acid is linked to an alcohol by an ester bond, compounds in which a carboxyl group in an amino acid is linked to an alcohol by an ester bond, and dimer acids and/or diesters of dimer diols.

Ester oils in which an acid is linked with a monohydric alcohol include, for example, isopropyl myristate, cetyl octanoate (cetyl ethylhexanoate), octyldodecyl myristate, isopropyl palmitate, 2-ethylhexyl palmitate, butyl stearate, hexyl laurate, myristyl myristate, decyl oleate, hexyldecyl dimethyl octanoate, lanolin acetate, isocetyl stearate, isocetyl isostearate, cholesteryl 12-hydroxystearate, castor oil fatty acid methyl ester, oleyl oleate, 2-heptylundecyl palmitate, ethyl laurate, 2-hexyldecyl myristate, 2-hexyldecyl palmitate, ethyl acetate, butyl acetate, amyl acetate and the like.

Ester oils in which an acid is linked with a polyhydric alcohol include, for example, ethylene glycol di-2-ethylhexanoate, dipentaerythritol fatty acid ester, n-alkylglycol monoisostearate, neopentyl glycol dicaprate, diisostearyl malate, glycerin di-2-heptyl undecanoate, trimethylolpropane tri-2-ethylhexanoate, trimethylolpropane triisostearate, pentaerythrityl tetraethylhexanoate, glyceryl tri-2-ethylhexylate, trimethylolpropane triisostearate, glycerin trimyristate, glyceride tri-2-heptylundecanoate, diisobutyl adipate, di-2-heptylundecyl adipate, di-2-ethylhexyl sebacate, 2-hexyldecyl adipate, diisopropyl sebacate, triethyl citrate and the like.

Ester oils in which a carboxyl group in an amino acid is linked to an alcohol by an ester bond include, for example, N-lauroyl-L-glutamic acid 2-octyldodecyl ester, di(phytosteryl/2-ocyltodecyl) N-lauroyl-glutamate, di(phytosteryl/behenyl/2-octyldodecyl) N-lauroyl-glutamate, di(cholesteryl/2-octyldodecyl) N-lauroyl-glutamate, di(cholesteryl/behenyl/2-octyldodecyl) N-lauroyl-glutamate, N-lauroyl-sarcosine isopropyl and the like.

Dimer acids and/or diesters of dimer diols include, for example, di(phytosteryl/isostearyl/cetyl/stearyl/behenyl) dimer dilinoleate, di(isostearyl/phystostearyl) dimer dilinoleate, dilinoleyl dimer dilinoleate, dimer dilinoleyl diisostearate, acetoglyceride and the like.

The ester oil blended into the cosmetic of the present invention may be of one type or a mixture of two or more types. In particular, an ester oil formed from a fatty acid and a polyhydric alcohol, for example, pentaerythrityl tetraethylhexanoate, is preferably used.

The oils constituting the oil (component (c)) in the cosmetic of the present invention aside from the ester oil are not particularly limited as long as they are oil-based components that are normally blended into cosmetics.

Specific examples include liquid oils/fats such as avocado oil, camellia oil, macadamia nut oil, mink oil, olive oil, castor oil and jojoba oil; solid oils/fats such as cacao butter, coconut oil, palm oil, palm kernel oil, beef tallow, pork tallow, mutton tallow, horse oil, hardened oils, hardened castor oil, Japan tallow and shea butter; waxes such as beeswax, candelilla wax, cotton wax, carnauba wax, tree wax, spermaceti, montan wax, rice bran wax, lanolin, reduced lanolin, hard lanolin, kapok wax, sugarcane wax, jojoba wax and shellac wax; hydrocarbons such as liquid paraffin, squalane, paraffin, ceresin and squalene; higher fatty acids such as lauric acid, myristic acid, palmitic acid, stearic acid, behenic aid, oleic acid, 12-hydroxystearic acid, isostearic acid, linolic acid and linoleic acid; higher alcohols such as lauryl alcohol, cetyl alcohol, stearyl alcohol, behenyl alcohol, oleyl alcohol, monostearyl glycerol ether, monopalmityl glycerol ether, cholesterol, phytosterol and isostearyl alcohol; and silicone oils. It is possible to use one type or a combination of two or more types of these oils.

The cosmetic of the present invention may be formulated so as not to contain, as the oil, an oil (solid oil) that is solid at ambient temperature (25 °C), such as a solid oil/fat or a wax. The cosmetic is preferably formulated so as not to contain a solid oil because there is then no need to provide a heating step in the production process, and the cosmetic can be prepared at ambient temperature.

Additionally, when (acrylates/(C10-30) alkyl acrylate) crosspolymer is used as the (b) alkyl-modified carboxyvinyl polymer, if a higher alcohol that is solid at ambient temperature is blended as the (c) oil, then there are cases in which gelling occurs and a stable oil-in-water emulsion cannot be obtained. Thus, the cosmetic of the present invention includes embodiments not containing a higher alcohol that is solid at ambient temperature.

The blended amount of the (c) oil in the cosmetic of the present invention should be 10% to 40% by mass, preferably 15% to 35% by mass, more preferably 20% to 30% by mass relative to the total amount of the cosmetic. If the blended amount of the (c) oil is less than 10% by mass, then a fitting sensation cannot be obtained when applied. If more than 40% by mass is blended, then the stability becomes lower and a watery feeling in use cannot be obtained.

The ester oil is essentially blended so as to constitute at least 50% by mass, preferably at least 60% by mass of the total amount of the oil. Although the upper limit value of the percentage of the ester oil relative to the total amount of the oil is not particularly limited, it may, for example, be 100% by mass or less, 90% by mass or less, or 80% by mass or less. If the percentage by mass of the ester oil relative to the total amount of the oil is less than 50% by mass, then the fitting sensation and the wateriness become inadequate.

In addition to the essential components (a) to (c) mentioned above, other components that can be blended into oil-in-water emulsion cosmetics may be blended, as appropriate, into the emulsion cosmetic of the present invention within a range not compromising the effects of the present invention. Specific examples include thickeners, humectants, neutralizers, various types of medicinal agents, various types of extracts, stabilizers, preservatives, chelating agents, masking agents, colorants, fragrances, essential oils and the like.

Since the cosmetic of the present invention can stably retain retinol, there is no need to substantially blend an antioxidant (for example, a phenolic antioxidant such as tocopherol or dibutylhydroxytoluene (BHT)) that was conventionally blended into conventional retinol-containing compositions. However, one may be blended, as appropriate, within a range not impacting the effects of the present invention.

Examples of thickeners include xanthan gum, tamarind gum, sodium alginate, gellan gum, agar, polyvinyl alcohol, quinceseed gum, non-alkyl-modified carboxyvinyl polymers (in the present specification, referred to simply as "carbomers") and the like. Commercially available products that are non-alkyl-modified carboxyvinyl polymers include, for example, CARBOPOL 941 polymer, CARBOPOL 980 polymer, CARBOPOL 981 polymer (manufactured by Lubrizol Advanced Materials), Synthalen K, Synthalen L (manufactured by 3V SIGMA) and the like.

Humectants include, for example, 1,3-butylene glycol (BG), polyethylene glycol, propylene glycol, dipropylene glycol (DPG), hexylene glycol, glycerin, diglycerin, xylitol, maltitol, maltose, D-mannitol, trehalose, erythritol, propanediol, glucose, polyoxyethylene methyl glucoside and the like.

Chelating agents include sodium edetic acid salts, sodium metaphosphate, phosphoric acid and the like.

The various types of medicinal agents include vitamins such as inositol, pyridoxine hydrochloride, benzyl nicotinate, nicotinic acid amide, dl-α-tocopherol nicotinate, magnesium ascorbyl phosphate, ascorbic acid 2-glucoside, 2-L ascorbic acid dl-α-tocopherol phosphoric acid diester potassium salt, pantothenic acid and biotin; anti-inflammatory agents such as allantoin and azulene; whiteners such as arbutin, 4-methoxysalicylic acid and salts thereof, tranexamic acid and derivatives thereof; saponifying agents such as tannic acid; lysozyme chloride, pyridoxine hydrochloride, γ-oryzanol, marine collagen, marine elastin and the like.

The various types of extracts include gambier extract, beech bud extract, turmeric extract, *Houttuynia cordata* extract, *Phellodendron amurense* extract, melilot extract, *Lamium album* extract, licorice extract, peony extract, *Saponaria officinalis* extract, loofah extract, quina extract, *Saxifraga stolonifera* extract, *Sophora angustifolia* extract, *Nuphar japonica* extract, *Sapindus mukorossi* extract, fennel extract, primrose extract, rose extract, *Rehmannia glutinosa* extract, lemon extract, *Lithospermum erythrorhizon* extract, aloe extract, *Thymus quinquecostatus* extract, eucalyptus extract, horsetail extract, sage extract, thyme extract, tea extract, *Rubus suavissimus* extract, seaweed extract, cucumber extract, clove extract, raspberry extract, *Melissa officinalis* extract, *Panax ginseng* extract, carrot extract, horse chestnut extract, peach extract, peach leaf extract, mulberry extract, cornflower extract, hamamelis extract, placental extract, thymus extract, silk extract, yeast extract and the like.

The emulsion cosmetic of the present invention may be produced in accordance with a conventionally used method. For example, it may be produced by separately mixing the oil phase components and the water phase components to respectively prepare the oil phase and the water phase, mixing the oil phase and the water phase, then emulsifying the mixture with a homomixer or the like.

The cosmetic of the present invention can stably retain retinol, which has wrinkle alleviation effects, and also has wateriness and an excellent fitting sensation when applied. By making use of these characteristics, the cosmetic of the present invention is suitable for being provided as an emulsion, a cream or a beauty lotion for use on the face, particularly around the eyes.

### EXAMPLES

Hereinafter, the present invention will be explained in further detail by providing examples. However, the present invention is not limited by these examples.

The amounts in the following examples and the like are expressed in percentage by mass where not particularly noted otherwise.

The oil-in-water emulsion cosmetics (samples) having the compositions indicated in Table 1 below were prepared, and the characteristics thereof were evaluated in accordance with the evaluation methods and evaluation criteria indicated below. The evaluation results are also indicated in Table 1.

### (1) Emulsion stability

Each sample was left for four weeks at 50 °C, then returned to room temperature, and the state of the sample was evaluated, by visual observation, in accordance with the criteria below.
A: Absolutely no oil separation was observed.
B: Slight oil separation was observed.
C: Clear separation was observed.

### (2) Actual usage tests of feeling in use

### • Wateriness

Eight expert panelists actually used each sample and judged the wateriness thereof.

### Evaluation criteria

S: Seven or eight out of the eight replied that there was a good texture with wateriness and freshness.
A: Five or six out of the eight replied that there was a good texture with wateriness and freshness.
B: Three or four out of the eight replied that there was a good texture with wateriness and freshness.
C: Two or more out of the eight replied that the texture was poor, with no sensation of wateriness.

### • Fitting sensation when applied

Eight expert panelists actually used each sample and judged the fitting sensation when applied.

### Evaluation criteria

S: Seven or eight out of the eight replied that there was a good texture with an excellent fitting sensation when applied.
A: Five or six out of the eight replied that there was a good texture with an excellent fitting sensation when applied.
B: Three or four out of the eight replied that there was a good texture with an excellent fitting sensation when applied.
C: Two or more out of the eight replied that the texture was poor, without an excellent fitting sensation when applied.

**[Table 1]**

| | Ex 1 | Comp Ex 1 | Comp Ex 2 | Comp Ex 3 |
|---|---|---|---|---|
| Purified water | balance | balance | balance | balance |
| Ethanol | 5 | 5 | 5 | 5 |
| Glycerin | 5 | 5 | 5 | 5 |
| BG | 5 | 5 | 5 | 5 |
| Sodium hydroxide | 0.15 | 0.15 | 0.15 | 0.15 |
| Carbomer | 0.25 | 0.25 | 0.25 | 0.25 |
| (Acrylates/(C10-30) alkyl acrylate) crosspolymer | 0.1 | 0.1 | 0.1 | 0.1 |
| Xanthan gum | 0.05 | 0.05 | 0.05 | 0.05 |
| Pentaerythrityl tetraethylhexanoate | 15 | 4 | 25 | 12 |
| Hydrogenated polydecene | 5 | 2 | 8 | 10 |
| Squalane | 5 | 2 | 8 | 5 |
| Retinol | 0.05 | 0.05 | 0.05 | 0.05 |
| Tranexamic acid | 2 | 2 | 2 | 2 |
| Sodium pyrosulfite | q.s. | q.s. | q.s. | q.s. |
| Phenoxy ethanol EDTA-3Na | q.s. | q.s. | q.s. | q.s. |
| | q.s. | q.s. | q.s. | q.s. |
| Fragrance | q.s. | q.s. | q.s. | q.s. |
| Total | 100 | 100 | 100 | 100 |
| Oil amount (%) | 25 | 8 | 41 | 27 |
| Percentage (%) of ester oil in total oil amount | 60 | 50 | 61 | 44 |
| Stability | A | A | B | A |
| Fitting sensation | A | C | A | B |
| Wateriness | A | S | C | B |

### Production method:

Each of the above-mentioned samples was substantially produced by the method indicated below.

The oil phase components (pentaerythrityl tetraethylhexanoate, hydrogenated polydecene, squalane and retinol) and the water phase components (components other than the above) were mixed separately from each other, then, while adding the oil phase components to the water phase components, the components were emulsified with an emulsifying machine to obtain the oil-in-water emulsion.

From the results indicated in Table 1, it was discovered that an emulsion cosmetic (Example 1) containing (acrylates/(C10-30) alkyl acrylate) crosspolymer as the (b) alkyl-modified carboxyvinyl polymer, and containing an oil in which an ester oil (pentaerythrityl tetraethylhexanoate) constituted at least 50% by mass (60% by mass) of the total amount of the oil, in an amount (25% by mass) within a prescribed range of the total amount of the cosmetic, had a good fitting sensation and provided a watery and fresh feeling in use.

Conversely, with a cosmetic (Comparative Example 1) in which the blended amount of the oil was 8% by mass, which is less than the prescribed range in the present invention, the fitting sensation when applied was significantly poorer. Additionally, a cosmetic (Comparative Example 2) in which the blended amount of the oil was 41% by mass, which exceeds the prescribed range in the present invention, lacked wateriness. Furthermore, with a cosmetic (Comparative Example 3) in which the amount of oil was within the range (27% by mass) prescribed in the present invention, but the percentage of ester oil relative to the total amount of the oil was 44% by mass (less than 50% by mass), the fitting sensation and the wateriness were both inadequate.

Formulation examples of oil-in-water emulsion cosmetics according to the present invention will be indicated below. However, the present invention is not limited to these formulations.

### Formulation Example 1:

| (Formulation) | (% by mass) |
|---|---|
| Purified water | balance |
| Alcohol | 6 |
| Glycerin | 3 |
| Butylene glycol | 7 |
| Potassium hydroxide | 0.2 |
| Carbomer | 0.2 |
| (Acrylates/(C10-30) alkyl acrylate) crosspolymer | 0.15 |
| Xanthan gum | 0.05 |
| Cetyl octanoate | 8 |
| Diisostearyl malate | 4 |
| Liquid paraffin | 6 |
| Retinol | 0.05 |
| Tranexamic acid | 1 |
| 4-Methoxysalicylic acid salt | 1 |
| Inositol | 0.1 |
| *Uncaria gambir* extract | 0.1 |
| Sodium pyrosulfite | 0.02 |
| Phenoxyethanol | 0.5 |
| EDTA-3Na | 0.1 |
| Fragrance | 0.2 |
| Total | 100 |

### Formulation Example 2:

| (Formulation) | (% by mass) |
|---|---|
| Purified water | balance |
| Alcohol | 3 |
| Glycerin | 6 |
| Butylene glycol | 4 |
| Potassium hydroxide | 0.25 |
| Carbomer | 0.3 |
| (Acrylates/(C10-30) alkyl acrylate) crosspolymer | 0.3 |
| (Dimethylacrylamide/sodium acryloyldimethyl taurate) crosspolymer | 0.05 |
| Octyl palmitate | 15 |
| Glyceryl tri-2-ethylhexanoate | 10 |
| Squalane | 10 |
| Retinol | 0.03 |
| Nicotinic acid amide | 5 |
| dl-Tocopherol acetate | 0.1 |
| Sodium hyaluronate | 0.01 |
| *Lamium album* extract | 0.1 |
| Licorice extract | 0.1 |
| Peony extract | 0.1 |
| Phenoxyethanol | 0.5 |
| Sodium hexametaphosphate | 0.1 |
| Total | 100 |

## Claims

1. An oil-in-water emulsion cosmetic comprisin:
(a) retinol;
(b) an alkyl-modified carboxyvinyl polymer; and
(c) an oil; wherein
an amount of the (c) oil is 10% to 40% by mass relative to a total amount of the cosmetic, and the (c) oil contains at least 50% by mass of an ester oil relative to a total amount of the oil.

2. The oil-in-water emulsion cosmetic according to claim 1, wherein the (b) alkyl-modified carboxyvinyl polymer is a copolymer of an olefinic unsaturated carboxylic acid monomer with a long-chain alkyl (meth)acrylate monomer represented by the following general formula (1): 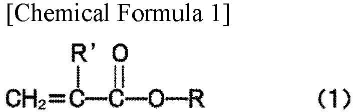 where R is a linear or branched alkyl group having 8 to 35 carbon atoms, and R' is hydrogen or a methyl group.

3. The oil-in-water emulsion cosmetic according to claim 2, wherein the (b) alkyl-modified carboxyvinyl polymer is a polymer of at least one type selected from (acrylates/(C10-30) alkyl acrylate) crosspolymer and (acrylates/(C12-22) alkyl methacrylate) copolymer.

4. The oil-in-water emulsion cosmetic according to claim 3, wherein the (b) alkyl-modified carboxyvinyl polymer is (acrylates/(C10-30) alkyl crosspolymer.

5. The oil-in-water emulsion cosmetic according to any one of claims 1 to 4, wherein the amount of the (b) alkyl-modified carboxyvinyl polymer is 0.01% to 4.0% by mass relative to the total amount of the cosmetic.

6. The oil-in-water emulsion cosmetic according to any one of claims 1 to 5, wherein the ester oil is a fatty acid ester of a polyhydric alcohol.

7. The oil-in-water emulsion cosmetic according to claim 6, wherein the fatty acid ester of a polyhydric alcohol includes pentaerythrityl tetraethylhexanoate.

8. The oil-in-water emulsion cosmetic according to any one of claims 1 to 7, not containing a solid oil.

9. The oil-in-water emulsion cosmetic according to any one of claims 1 to 8, containing substantially no antioxidants.
